# EUROPEAN PATENT APPLICATION

(11) **EP 3 777 672 A1**
(43) Date of publication of application: **17.02.2021**
(21) Application number: 19775350.2
(22) Date of filing: 22.02.2019
(51) Int. Cl.: A61B 5/042, A61B 5/0472, A61M 25/00, A61M 25/01

(54) **CAPTURE CATHETER FOR SENSING MYOCARDIAL ELECTRICAL SIGNAL**

(30) Priority: 26.03.2018 KR 20180034716
(71) Applicant: Taupnu Medical Co., Ltd., Busan 46241 (KR)
(72) Inventor: KIM, June Hong, Busan 48516 (KR); CHON, Minku, Yangsan-si Gyeongsangnam-do 50658 (KR); KIM, Junoh, Yangsan-si Gyeongsangnam-do 50595 (KR); JUNG, Sujin, Yangsan-si Gyeongsangnam-do 50601 (KR)
(74) Representative: HGF
(86) International application number: PCT/KR2019/002193
(87) International publication number: WO 2019/190060

(57) **Abstract**

A capture catheter for sensing myocardial electrical signals is proposed, the capture catheter sensing the His bundle by sensing myocardial electrical signals and capturing a rope having passed through the His bundle. The capture catheter includes: a sheath catheter having a through hole formed therein; a myocardial sensing catheter inserted into the through hole of the sheath catheter to sense the His bundle; and at least one capturing device of which both ends are coupled to an outer circumferential surface of the myocardial sensing catheter to form an annular shape.

## Description

### Technical Field

The present invention relates to a capture catheter for sensing myocardial electrical signals and, more particularly, to a capture catheter for sensing myocardial electrical signals, the capture catheter sensing the His bundle by sensing myocardial electrical signals and capturing a rope having passed through the His bundle.

### Background Art

The cardiac conduction system is configured so that electrical signals arising in the sinoatrial node in the atrium travel to the atrioventricular node through the His bundle, down the bundle branches (left and right His bundles), and to the respective Purkinje fibers, causing the ventricles to contract.

In an electrocardiogram, a QRS wave is generated through the depolarization process of the ventricular muscle, the first downward wave following a P wave is referred to as a Q wave, the first upward wave is referred to as an R wave, and the downward wave following the R wave is referred to as an S wave. A width of the QRS wave refers to the time that electricity is conducted from the His bundle to the entire ventricle. The width of the QRS wave is normally within about 0.12 seconds (about 90 ms). When the width is 0.12 seconds (120ms) or more, it means there is a conduction disorder in the ventricle. When the electric conduction time is long, the width of QRS becomes wider, and when the electric conduction time is short, the width of QRS becomes narrower. A wider width of QRS causes ventricular dyssynchrony which makes a ventricular motion asynchronous, so that there is a side effect that is a loss of ventricular function.

When dysfunction occurs in the cardiac conduction system itself, or when electricity is abnormally generated in the cardiac conduction system and the electricity is transferred to another place, arrhythmia occurs.

As a method of diagnosing patients with cardiac arrhythmia disease, there are an electrocardiogram, Holter monitoring, electrophysiological tests, and the like.

Among these, the electrophysiological test measures and records electrical activity in various parts of the heart, or observes the reaction of the heart by directly applying electrical stimulation to various parts of the heart, in a state in which a thin catheter equipped with electrodes is inserted into the heart through a blood vessel in the arm or leg. Through the electrophysiological test, a doctor may be able to pinpoint the exact area where the arrhythmia occurs and perform a surgical procedure to remove the discovered area, or treat the arrhythmia using an artificial pacemaker.

As a part of this study, the present inventor proposed a method, apparatus, and catheter for positioning the end of the pacemaker lead passing through the coronary sinus in the ventricular septum, which is published in Korean Patent Application Publication No. 10-2016-001530 and Korean Patent Application Publication No. 10-2016-0020887. The above patent applications are invented to improve the rope in Korean Patent No. 10-1116867 proposed by the present inventor and relate to a device capable of fixing the artificial pacemaker to the heart more easily, as compared to the related art.

However, when treating the arrhythmia using the artificial pacemaker, applying electrical stimulation to an area that is near the conduction system located in the ventricular septum shortens an electrical conduction time and makes a width of the QRS narrow. Therefore, there is a need for research on a device that can be safely and conveniently fixed to the heart while applying electrical stimulation to the area that is near the conduction system.

### Disclosure

### Technical Problem

An objective of the present invention is to provide a capture catheter for sensing myocardial electrical signals, the capture catheter being capable of easily sensing myocardial electrical signals and capturing a rope having passed through the His bundle in a cerclage surgical procedure.

In addition, the present invention provides a capture catheter for sensing myocardial electrical signals, the capture catheter capable of being applied to a variety of electrophysiology (EP) catheters commonly used.

The objectives of the present invention are not limited to the above-mentioned objectives, and other objectives that are not mentioned will be clearly understood by those of ordinary skill in the art from the following description.

### Technical Solution

The capture catheter for sensing myocardial electrical signals according to the present invention includes a sheath catheter having a through hole formed therein; a myocardial sensing catheter inserted into the through hole of the sheath catheter to sense the His bundle; and at least one capturing device of which both ends are coupled to an outer circumferential surface of the myocardial sensing catheter to form an annular shape.

The myocardial sensing catheter may have a plurality of electrodes coupled to an upper portion thereof to sense the myocardial electrical signals, and the capturing device may be folded when being inserted into the sheath catheter and may be unfolded when being removed from the sheath catheter.

In addition, the myocardial sensing catheter may have a positioning device coupled to a lower portion thereof, so that an upper portion of the myocardial sensing catheter moves by adjusting the positioning device.

The capture catheter for sensing myocardial electrical signals according to another embodiment of the present invention includes a sheath catheter having a through hole formed therein; an inner catheter inserted into the through hole of the sheath catheter and having a through hole formed therein; a myocardial sensing catheter inserted into the through hole of the inner catheter to sense the His bundle; and at least one capturing device of which both ends are coupled to an outer circumferential surface of the inner catheter to form an annular shape.

The myocardial sensing catheter may have a plurality of electrodes coupled to an upper portion thereof to sense the myocardial electrical signals, and the capturing device may be folded when being inserted into the sheath catheter and may be unfolded when being removed from the sheath catheter.

In addition, the myocardial sensing catheter may have a positioning device coupled to a lower portion thereof, so that an upper portion of the myocardial sensing catheter moves by adjusting the positioning device.

The inner catheter may be provided in such a manner as to be detachable from the myocardial sensing catheter.

### Advantageous Effects

The capture catheter for sensing myocardial electrical signals according to the present invention can easily sense myocardial electrical signals to capture the rope having passed through the His bundle in a cerclage surgical procedure.

In addition, the capture catheter for sensing myocardial electrical signals according to the present invention can be applied to a variety of electrode catheters commonly used.

### Description of Drawings

FIG. 1 is a perspective view showing a capture catheter for sensing myocardial electrical signals according to a preferred embodiment of the present invention.
FIG. 2 is a perspective view showing a capture catheter for sensing myocardial electrical signals according to another preferred embodiment of the present invention.
FIG. 3 is a flow chart showing a method of capturing a rope with a catheter for sensing myocardial electrical signals according to the present invention.

### <Description of reference numerals in drawings>

- 10 :: sheath catheter
- 20 :: myocardial sensing catheter
- 22 :: electrode
- 24 :: positioning device
- 30 :: capturing device
- 32 :: junction fastener
- 40 :: inner catheter
- 50 :: guide wire
- 60: : rope

### Mode for Invention

Advantages and features of the present invention, and a method of achieving them will become apparent with reference to the embodiments described below in detail together with the accompanying drawings. However, the present invention is not limited to the embodiments disclosed below, and may be implemented in various different forms. The embodiments are provided to complete the disclosure of the present invention, and to completely inform the scope of the invention to those of ordinary skill in the art to which the present invention belongs, and the invention is only defined by the scope of the claims.

With reference to the accompanying drawings, the implementation of the present invention will be described in detail. Regardless of the drawings, the same reference numerals refer to the same elements, and "and/or" includes each of the mentioned items and all combinations of one or more.

The terms used in this specification are for describing exemplary embodiments, and are not intended to limit the present invention. In this specification, the singular form also includes the plural form unless specifically stated in the phrase. As used herein, "comprises" and/or "comprising" do not exclude the presence or addition of one or more other elements other than the mentioned elements.

Unless otherwise defined, all terms (including technical and scientific terms) used in the present specification may be used as meanings that can be commonly understood by those of ordinary skill in the art to which the present invention belongs. In addition, terms defined in a commonly used dictionary are not interpreted ideally or excessively unless specifically explicitly defined.

Hereinafter, preferred embodiments of the present invention will be described in detail with reference to the accompanying drawings.

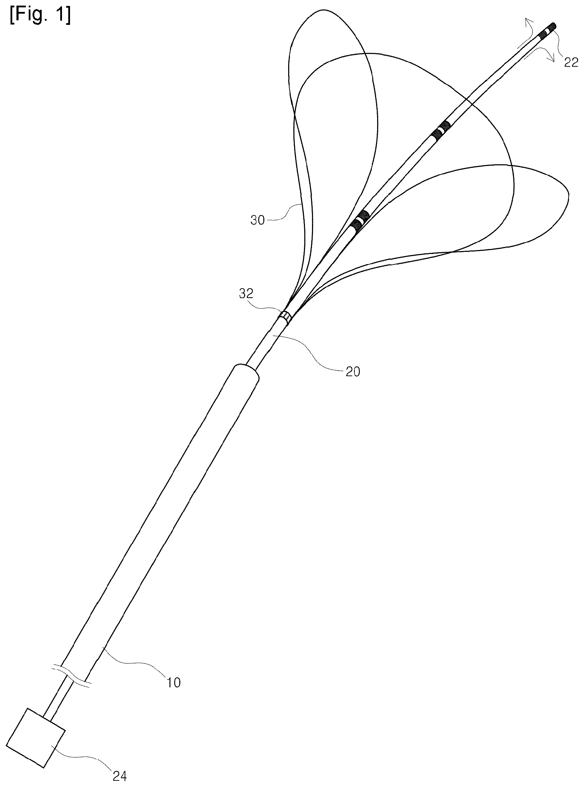

FIG. 1 is a perspective view showing a capture catheter for sensing myocardial electrical signals according to a preferred embodiment of the present invention.

Referring to FIG. 1, the myocardial electrical signal sensing catheter according to a preferred embodiment of the present invention includes a sheath catheter 10, a myocardial sensing catheter 20, and a capturing device 30.

The sheath catheter 10 is formed with a hollow cylinder having a through hole therein, and is composed of synthetic resin materials, such as a soft rubber material, a soft plastic material, and the like, which are materials that are soft and are excellent in flexibility and resilience.

The myocardial sensing catheter 20 includes an electrode 22 coupled to an upper portion and a positioning device 24 coupled to a lower portion. The electrode 22 serves to sense electrical signals of the myocardium, and one or more electrodes may be included. The electrode senses the electrical signals of the myocardium to find the location of the His bundle. The myocardial sensing catheter 20 may be an electrophysiology (EP) catheter commonly used.

The myocardial sensing catheter 20 may be configured so that an upper portion thereof moves by adjusting the positioning device 24. As the upper portion moves, the electrode 22 coupled to the upper portion contacts various positions of the myocardium while moving, thereby sensing electrical signals. The myocardial electrical signal sensed through the electrode 22 may be checked through an external recording device. Herein, the electric signals of the His bundle are sensed to identify the location of the His bundle, and a rope 60 is allowed to pass through the His bundle.

The capturing device 30 has both ends coupled to an outer circumferential surface of the myocardial sensing catheter 20 to form an annular shape, and has a shape of a snare or loop. A plurality of capturing devices, preferably three or four capturing devices, may be included. The capturing device 30 may be made of a metal material (SUS304, Nitinol, Titanium, etc.) capable of being inserted into the human body, and may be a shape memory alloy, elastic body, or a self-expandable stent.

A junction fastener 32 is included to combine the plurality of capturing devices 30, and the junction fastener 32 is provided in such a manner as to cover both ends of the plurality of capturing devices 30 coupled to the outer peripheral surface of the myocardial sensing catheter 20.

As the myocardial sensing catheter 20 is moved vertically, the capturing device 30 is inserted into the sheath catheter 10 and thus folded, or removed from the sheath catheter 10 and thus unfolded. The rope 60 is located in the capturing device 30 after having passed through the His bundle, and the myocardial sensing catheter 20 is pulled downward so that the capturing device 30 is inserted into the sheath catheter 10, thereby capturing the rope 60.

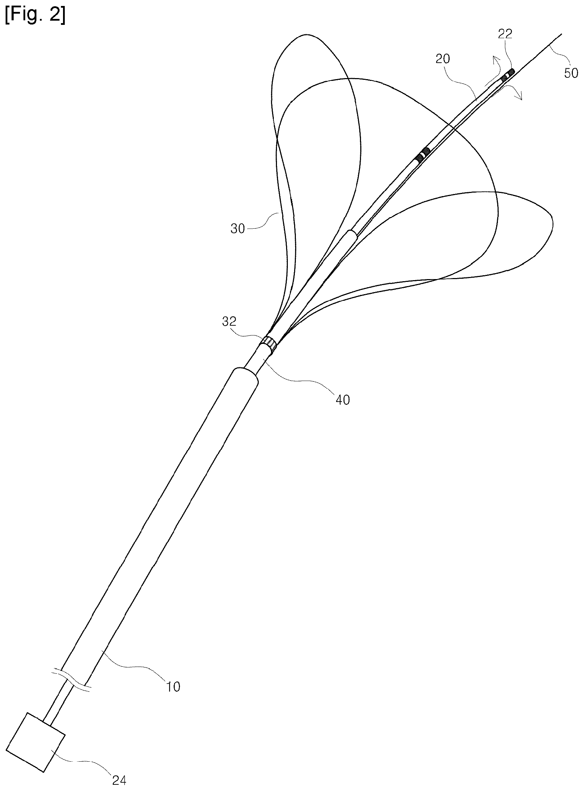

FIG. 2 is a perspective view showing a capture catheter for sensing myocardial electrical signals according to another preferred embodiment of the present invention.

Referring to FIG. 2, a capture catheter for sensing myocardial electrical signals according to another preferred embodiment of the present invention includes a sheath catheter 10, a myocardial sensing catheter 20, a capturing device 30, and an inner catheter 40.

The sheath catheter 10 is formed with a hollow cylinder having a through hole therein, and composed of synthetic resin materials, such as soft rubber material, soft plastic material, and the like, which are materials that are soft and are excellent in flexibility and resilience.

The inner catheter 40 is inserted into the through hole of the sheath catheter 10, and a plurality of capturing devices 30 are coupled to an outer circumferential surface of the inner catheter 40.

The capturing device 30 has both ends coupled to an outer circumferential surface of the myocardial sensing catheter 20 to form an annular shape, and has a shape of a snare or loop. The plurality of capturing devices, preferably three or four capturing devices may be included. The capturing device 30 may be made of a metal material (SUS304, Nitinol, Titanium, etc.) capable of being inserted into the human body, and may be a shape memory alloy, elastic body, or a self-expandable stent.

A junction fastener 32 is included to combine the plurality of capturing devices 30, and the junction fastener 32 is provided in such a manner as to cover both ends of the plurality of capturing devices 30 coupled to the outer peripheral surface of the inner catheter 40.

The inner catheter 40 moves vertically to be inserted into or removed from the sheath catheter 10, so that the capturing device 30 may be inserted into or removed from the sheath catheter 10.

The inner catheter 40 has a through hole formed therein, and the myocardial sensing catheter 20 is inserted into the through hole of the inner catheter 40. The myocardial sensing catheter 20 includes an electrode 22 coupled to an upper portion thereof and a positioning device 24 coupled to a lower portion thereof. The electrode 22 serves to sense electrical signals of the myocardium, and one or more electrodes may be included. Herein, the electrode may find the location of the His bundle by sensing the electrical signals of the myocardium.

The inner catheter 40, to which the capturing device 30 is coupled, may be used in combination with various electrophysiology (EP) catheters commonly used, if necessary.

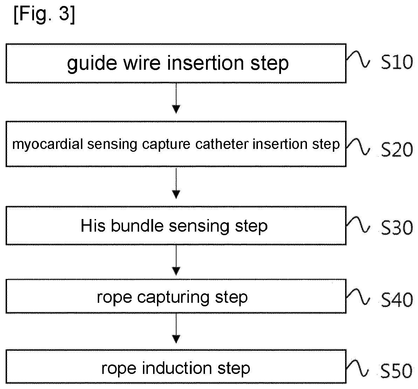

FIG. 3 is a flow chart showing a method of capturing a rope with a catheter for sensing myocardial electrical signals according to the present invention.

Referring to FIG. 3, in a guide wire insertion step (S10), a guide wire 50 is inserted into a human body.

Next, in a myocardial sensing capture catheter insertion step (S20), the myocardial sensing catheter 20 and the capturing device 30 coupled to the myocardial sensing catheter 20 are inserted into the inner through hole of the sheath catheter 10, and the guide wire 50 is inserted into the sheath catheter 10, whereby the sheath catheter is inserted into the human body to be moved to the right ventricle according to the guide wire 50.

Next, in a His bundle sensing step (S30), the myocardial sensing catheter 20 is pushed upward, so that the myocardial sensing catheter 20 and the capturing device 30 are removed to the outside, and then an electrode 22 coupled to the myocardial sensing catheter 20 senses electrical signals of the myocardium, and a positioning device 24 coupled to the rear end of the myocardial sensing catheter 20 is controlled to sense the electrical signals from various places in the myocardium, thereby finding the location of the His bundle.

In a rope capturing step (S40), when the rope 60 having passed through the His bundle is located in the capturing device 30, the myocardial sensing catheter 20 is pulled downward so that the capturing device 30 is inserted into the sheath catheter 10 together with the rope 60.

Finally, in a rope induction step (S50), the capture catheter according to the present invention captures the rope having passed through the His bundle and moves the rope to the inferior vena cava or superior vena cava, thereby inducing the rope.

Although the embodiments of the present invention have been described with reference to the above and the accompanying drawings, those of ordinary skill in the art to which the present invention pertains will be able to understand that the present invention can be implemented in other specific forms without changing the technical spirit or essential features. Therefore, it is to be understood that the embodiments described above are illustrative in all respects and not limiting.

## Claims

1. A capture catheter for sensing myocardial electrical signals, the capture catheter comprising:
a sheath catheter having a through hole formed therein;
a myocardial sensing catheter inserted into the through hole of the sheath catheter to sense the His bundle; and
at least one capturing device of which both ends are coupled to an outer circumferential surface of the myocardial sensing catheter to form an annular shape.

2. The capture catheter of claim 1, wherein the myocardial sensing catheter has a plurality of electrodes coupled to an upper portion thereof to sense the myocardial electrical signals.

3. The capture catheter of claim 1, wherein the capturing device is folded when being inserted into the sheath catheter, and is unfolded when being removed from the sheath catheter.

4. The capture catheter of claim 1, wherein the myocardial sensing catheter has a positioning device coupled to a lower portion thereof, so that an upper portion of the myocardial sensing catheter moves by adjusting the positioning device.

5. A capture catheter for sensing myocardial electrical signals, the capture catheter comprising:
a sheath catheter having a through hole formed therein;
an inner catheter inserted into the through hole of the sheath catheter and having a through hole formed therein;
a myocardial sensing catheter inserted into the through hole of the inner catheter to sense the His bundle; and
at least one capturing device of which both ends are coupled to an outer circumferential surface of the inner catheter to form an annular shape.

6. The capture catheter of claim 5, wherein the myocardial sensing catheter has a plurality of electrodes coupled to an upper portion thereof to sense the myocardial electrical signals.

7. The capture catheter of claim 5, wherein the capturing device is folded when being inserted into the sheath catheter, and is unfolded when being removed from the sheath catheter.

8. The capture catheter of claim 5, wherein the myocardial sensing catheter has a positioning device coupled to a lower portion thereof, so that an upper portion of the myocardial sensing catheter moves by adjusting the positioning device.

9. The capture catheter of claim 5, wherein the inner catheter is provided in such a manner as to be detachable from the myocardial sensing catheter.
